# EUROPEAN PATENT SPECIFICATION

(11) **EP 1 699 493 B1**
(45) Date of publication and mention of the grant of the patent: **28.01.2009**
(21) Application number: 04821045.4
(22) Date of filing: 17.11.2004
(51) Int. Cl.: A61K 49/04, A61M 25/01

(54) **POLYMERIC MARKER WITH HIGH RADIOPACITY FOR USE IN MEDICAL DEVICES**
POLYMERER MARKER MIT HOHER RADIOPAZITÄT ZUR VERWENDUNG IN MEDIZINPRODUKTEN
MARQUEUR POLYMERE A RADIO-OPACITE ELEVEE A UTILISER DANS DES DISPOSITIFS MEDICAUX

(30) Priority: 29.12.2003 US 748016
(43) Date of publication of application: 13.09.2006
(73) Proprietor: Abbott Cardiovascular Systems Inc., Santa Clara, CA 95054 (US)
(72) Inventor: BAVARO, Vincent P., Temecula, CA 92592 (US); SIMPSON, John A., Carlsbad, CA 92009 (US); D'AQUANNI, Peter J., Murrieta, CA 92562 (US); BALDWIN, Aaron, Temecula, CA 92591 (US); CORNISH, Wayne E., Fallbrook, CA 92028 (US)
(74) Representative: McLeish, Nicholas Alistair Maxwell
(86) International application number: PCT/US2004/038610
(87) International publication number: WO 2005/065725

(56) References cited:
- EP-A- 0 203 833
- EP-A- 0 938 879
- EP-A- 0 987 042
- WO-A-93/10440
- US-A- 4 714 721
- US-A- 5 667 767
- US-A1- 2004 220 549
- US-B1- 6 355 058
- US-B1- 6 540 721

## Description

### BACKGROUND OF THE INVENTION

The present invention is directed to elongated intracorporeal devices, and more particularly intraluminal devices for stent deployment, percutaneous transluminal coronary angioplasty (PTCA), and the similar procedures. PTCA is a widely used procedure for the treatment of coronary heart disease. In this procedure, a balloon dilatation catheter is advanced into the patient's coronary artery and the balloon on the catheter is inflated within the stenotic region of the patient's artery to open up the arterial passageway and increase the blood flow through the artery. To facilitate the advancement of the dilatation catheter into the patient's coronary artery, a guiding catheter having a preshaped distal tip is first percutaneously introduced into the cardiovascular system of a patient by the Seldinger technique through the brachial or femoral arteries. The catheter is advanced therein until the preshaped distal tip of the guiding catheter is disposed within the aorta adjacent the ostium of the desired coronary artery. A balloon dilatation catheter may then be advanced through the guiding catheter into the patient's coronary artery until the balloon on the catheter is disposed within the stenotic region of the patient's artery.

Once properly positioned across the stenosis, the balloon is inflated one or more times to a predetermined size with radiopaque liquid at relatively high pressures (e.g., generally 4.05-12.16 x 10⁵ Pa (4-12 atmospheres) to dilate the stenosed region of a diseased artery. After the inflations, the balloon is finally deflated so that the dilatation catheter can be removed from the dilatated stenosis to resume blood flow.

Similarly, balloon catheters may be used.to deploy endoprosthetic devices such as stents. Stents are generally cylindrical shaped intravascular devices that are placed within a damaged artery to hold it open. The device can be used to prevent restenosis and to maintain the patency of blood vessel immediately after intravascular treatments. Typically, a compressed or otherwise reduced diameter stent is disposed about an expandable member such as a balloon on the distal end of the catheter, and the catheter and stent thereon are advanced through the patient's vascular system. Inflation of the balloon expands the stent within the blood vessel. Subsequent deflation of the balloon allows the catheter to be withdrawn, leaving the expanded stent within the blood vessel.

Topically, the distal section of a balloon catheter or other percutaneous device will have one or more radiopaque markers in order for the operator of the device to ascertain its position and orientation under X-ray or fluoroscopy imaging. Generally, a band or ring of solid radiopaque metal is secured about an inner or outer shaft of a balloon catheter to serve as a radiopaque marker. Such configuration, however, locally stiffens the catheter shaft and thereby imparts an undesirable discontinuity thereto as the solid metal bands are relatively inflexible compared to a polymer balloon catheter shaft. Additionally, the metallic markers are relatively expensive to manufacture and relatively difficult to positively affix to an underlying device.

As is described in U.S. Patent No. 6,540,721 (Voyles, et al.), many of the problems associated with the use of conventional markers may be overcome by replacing the rigid precious metal tubing with a polymer that is filled or doped with a suitable radiopaque agent. Such marker may be formed by blending a polymer resin with a powdered, radiographically dense material such as elemental tungsten and then extruding the composition to form a tubular structure with an appropriate inner diameter and wall thickness. The extrusion may then be cut to discrete lengths and installed onto the intended component via a melt bonding process.

EP 0 203 833 disloses a radiopaque thermoset polymer and US 47 147 21 discloses composite materials comprising a plastic matrix and an inorganic radiopaque filler*.*

A shortcoming of such an approach has been found to be the apparent limit to which a suitable polymer can be filled with a radiographically dense material to yield a composition that can be successfully compounded, economically shaped into suitable dimensions for markers and easily assembled onto a component without unduly compromising the desirable properties of the polymer matrix. The fill ratio that is achievable will determine how thick a marker must be in order to achieve a particular degree of radiopacity. In the case of tungsten in a polymer such as Pebax, the fill ratio limit has heretofore been found to be about 80 weight percent. Such weight percentage equates to about 18 volume percent which requires the marker to be excessively thick in order to achieve adequate radiopacity.

A polymeric marker is therefore needed having a substantially higher fill ratio than has heretofore been possible. Such marker would allow devices to be rendered highly visible without an inordinate increase in overall profile nor a compromise of the flexibility of the underlying component.

### SUMMARY OF THE INVENTION

The present invention overcomes the shortcomings of previously described polymeric radiopaque markers by enabling a polymer to be filled or doped with a considerably greater quantity of a radiopaque agent than has heretofore been achievable. The increased fill ratio nonetheless allows uniform pellets to be compounded and an extrusion with the appropriate wall thickness to be formed. The resulting marker provides an unprecedented combination of radiopacity and flexibility. Such marker would allow any of various intraluminal devices to be radiopaquely marked including, but not limited to, coronary, peripheral, and guiding catheters as well as guide wires.

The marker of the present invention relies on the use of radiopaque materials with a preselected particle shape and a preselected particle size distribution as well as the inclusion of one or more additives in the polymer/radiopaque agent blend. The multifunctional polymeric additive maleic anhydride graft polyolefin (MA-g-PO) is added to the composition in order to enhance the wetting, adhesive and flow properties of the individual radiopaque particles by the polymer so as to cause each particle to be encapsulated by the polymer and thereby allow the polymer to form a continuous binder. An antioxidant may optionally be added in order to preserve the high molecular weight of the polymer matrix as it is exposed to the high temperatures and shear stresses associated with the compounding and extrusion processes.

While previous attempts to increase fill ratios have involved tungsten powder of relatively fine particle size, the present invention relies on the use of particles of increased size in order to achieve such end. An increase in particle size has been found to allow the polymer to more effectively function as a continuous binder and thereby increase ductility at a given fill ratio or maintain ductility at increased fill ratios. It has been found that in constraining the average particle size to at least 2 microns and limiting maximum particle size to about 20 microns provides the desired results. In the case of tungsten in Pebax, a fill ratio of about 91.3 weight percent (equivalent to 36.4 volume percent) is readily attainable.

In various alternative embodiments, an increase in the fill ratio of radiopaque particles in polymer up to about 95 weight percent is possible. Polymers capable of achieving the 95 weight percent loading ratio include Pebax, polyurethanes, nylon, polyetherurethanes, polyester copolymers, olefin derived copolymers, natural rubbers, synthetic rubbers, thermoplastic elastomers, and specialty polymers.

Increasing the radiopaque material weight percent in the polymer increases the density of the marker. A higher density marker benefits from several advantages over the prior art including increased radiopacity for a given size, and consequently the ability of the marker to be made with a smaller cross-sectional size improving crossing profile yet maintaining the same level of radiopacity. Also, a higher concentration of radiopaque material allows the marker band to be optionally made to a shorter length thereby minimizing any effects on flexibility of the stent, catheter, guide wire, or like device to which it is attached.

Additionally, it has been found that the process by which the tungsten powder is produced has a considerable effect on both particle size distribution as well as the shape of the individual particles. Tungsten powder produced by either a "pusher" process or "atomization" process, then milled and classified has been found to provide discrete particles having a more equiaxed shape and size respectively and are therefore more ideally suited for the purposes of the present invention than powders produced employing a "rotary" process.

The marker of the present invention is manufactured by first tumble mixing the polymer resin with the pelletized wetting agent, such as maleic anhydride graft polyolefin resin (MA-g-PO), and an antioxidant and then introducing the mixture into the primary feeder of a twin screw extruder. The mixture is fed in at a controlled mass flow rate and conveyed down the barrel length as it is heated above its melting temperature and blended. At a point downstream, tungsten powder is introduced into the mix at a controlled mass flow rate via a secondary feeder. The tungsten powder and the molten ingredients become intimately intermixed as they are conveyed downstream and discharged through a die as molten strands which are cooled in water and subsequently pelletized. The markers are subsequently formed by extruding the tungsten filled polymer onto a continuous beading of PTFE and drawn down to yield the desired wall thickness. The extrusion is then cut to the desired lengths, preferably with the beading still in place so as to provide support. Removal of the beading remnant then allows the marker to be slipped onto the medical device or component thereof to be marked and melt bonded in place. Reliance on melt bonding obviates the need for the marker to completely surround the underlying device. Markers can for example be longitudinally split in half to form two markers of C-shaped cross-section. Or, solid strands of extruded marker material may be melt bonded to one side to form one or more longitudinal stripes or helical patterns.

Due to its high radiopacity, flexibility and melt bondability, the marker of the present invention is readily attached to for example the inner member of a balloon catheter, a guide wire, and even a guide catheter tip. The attachment of radiopaque markers of known dimensions to a guide wire or the attachment to a guide wire of multiple radiopaque markers with known separation distances impart a measurement capability to the catheter that allows a physician to quickly and easily measure lesions and decide on appropriate stent lengths.

These and other features of the present invention will become apparent from the following detailed description of preferred embodiments which, taken in conjunction with the accompanying drawings, illustrate by way of example the principles of the present invention.

### Brief Description of the Drawings

Figure 1 is an enlarged side view of the radiopaque markers of the present invention attached to a balloon catheter;
Figure 2 is an enlarged side view of radiopaque markers of the present invention attached to a guide wire in a preferred configuration;
Figure 3 is an enlarged side view of the radiopaque markers of the present invention attached to a guide wire in an alternatively preferred configuration.
Figure 4 is a simplified, side elevational view of yet another embodiment of the present invention radiopaque markers on a guide wire; and
Figure 5 is a simplified, cross-sectional view of still another embodiment of the present invention radiopaque markers on a guide wire.

### Detailed Description of the Preferred Embodiments

The present invention provides a radiopaque polymer marker for use on a variety of devices that is flexible, highly radiopaque and is easily attachable to such devices by melt bonding. These properties allow markers to be of minimal thickness and thereby minimize the effect the marker has on the overall profile and stiffness of the device to which it is to be attached. Furthermore, the higher weight percent further increases the marker's radiopacity and allows a decrease in the marker's section size.

In order to achieve the high fill ratios that are necessary to attain the desired radiopacity and in order to do so without compromising the compoundability and workability of the polymeric material nor its ultimate strength and flexibility, a number of different parameters have been found to be of importance. More specifically, both the particle shape and particle size of the radiopaque agent must be carefully controlled while the inclusion of a MA-g-PO in the polymer blend is critical. An antioxidant may additionally be included in an effort to reduce the adverse effect the high processing temperatures and shear stresses may have on polymer properties.

A number of polymeric materials are well suited for use in the manufacture of the markers of the present invention. The material preferably comprises a low durometer polymer in order to render the marker sufficiently flexible so as not to impair the flexibility of the underlying medical device component to which the finished marker is to be attached. Additionally, the polymer must be compatible with the material of which the component is constructed so as to allow the marker to be melt bonded in place. The polymer must also impart sufficient strength and ductility to the marker compound so as to facilitate its extrusion and forming into a marker, its subsequent handling and attachment to a medical device and preservation of the marker's integrity as the medical device is flexed and manipulated during use. Examples of such polymers include but are not limited to polyamide copolymers like Pebax, polyetherurethanes like Pellethane, polyester copolymers like Hytrel, olefin derived copolymers, natural and synthetic rubbers like silicone and Santoprene, thermoplastic elastomers like Kraton and specialty polymers like EVA and ionomers, etc. as well as alloys thereof. Further acceptable polymers include nylon and polyurethanes. The preferred polymer for use in the manufacture of a marker in accordance with the present invention is polyether block polyamide copolymer. A durometer of 25 or lower is preferred.

A number of different metals are well known to be radiographically dense and can be used in a pure or alloyed form to mark medical devices so as to render them visible under fluoroscopic inspection. Commonly used metals include but are not limited to platinum, gold, iridium, palladium, rhenium and rhodium. Less expensive radiopaque agents include tungsten, tantalum, silver and tin, of which tungsten is most preferred for use in the markers of the present invention.

The control of particle size has been found to be of critical importance for achieving the desired ultra high fill ratios. While efforts to increase fill ratios have previously utilized small average particle sizes (1 micron or less) so as to minimize the ratio of particle size to as-extruded wall thickness, it has been found that higher fill percentages can be realized with the use of somewhat larger average particles sizes. It is desirable in the formulation of high fill ratio compounds to have the following attribute: 1) uniform distribution of the filler particles, 2) continuity of the surrounding polymer matrix, and 3) sufficient spacing between filler particles so that the polymer matrix provides ductility to the bulk mixture to impart processability in both the solid and molten state.

The use of larger average particle sizes results in greater spacing between filler particles at a given percentage, thus maintaining processability during compounding and especially subsequent extrusion coating. The upper limit of average particle size is determined by the wall thickness of the coating and the degree of non-uniformity tolerable (i.e., surface defects). It has been found that a particle size distribution having an average particle size range of at least 2 microns to 10 microns and a maximum particle size of about 20 microns yields the desired fill ratio and provides for a smooth surface in the marker made therefrom.

The control of particle shape has also been found to be of critical importance for achieving the desired ultra high fill ratios. Discrete particles of equiaxed shape have been found to be especially effective, as individual particles of irregular shape, including agglomerations of multiple particles, have been found to adversely impact the surface, and thus, the maximum fill ratio that is attainable.

It has also been found that the process by which certain metal powders are produced has a profound effect on the shape of the individual particles. In the case of metallic tungsten, the powders may be formed by the reduction of powdered oxides through either "rotary," "pusher" or "atomization" processing. Of these processes, "rotary" processing has been found to yield the least desirable shape and size distribution as partial sintering causes coarse agglomerates to be formed which do not break up during compounding or extrusion and thus adversely effect the marker manufactured therefrom. Atomized powders have been reprocessed by melting and resolidifying "rotary" or "pusher" processed powders and result in generally equiaxed, discrete particles which are suitable for use in the present invention. "Pusher" processed powders are preferred due to their low cost and discrete, uniformly shaped particles.

In order for the polymer to most effectively encapsulate individual radiopaque particles, it is necessary for a low-energy interface to exist between such particles and the polymer so as to enable the polymer to "wet" the surface of the particles. Certain additives such as surfactants and coupling agents may serve as wetting agents and adhesion promoters for polymer/metal combinations that are not naturally compatible. It has been found that additives containing maleic anhydride graft to a polyolefin backbone provide a significant benefit in this regard wherein materials commercially available as Lotader 8200 (having LLOPE Backbone) and Licomont AR504 (having PP backbone) were found to be particularly effective for use with tungsten/Pebax combinations. Emerging extrusions were found to be less susceptible to breakage and the melt viscosity during compounding was lower as was manifested by a reduction in torque exerted during the extrusion process. The use of such additives allowed compounds with higher fill ratios to be successfully produced.

The inclusion of an antioxidant in the marker composition has also been found to be of benefit. A commercially available antioxidant such as Irganox B225 have been found to minimize degradation (i.e., reduction in molecular weight) of the polymer matrix as it is exposed to the multiple heat and shear histories associated with the compounding, extrusion, and bonding processes.

The compound used for the manufacture of the marker of the present invention is preferably made by first blending the polymer resin and wetting agent, and optionally, an antioxidant such as by tumble mixing after which such blend is introduced into a twinscrew extruder via a primary feeder. The feed rate is carefully controlled in terms of mass flow rate to ensure that a precise fill ratio is achieved upon subsequent combination with the radiopaque agent. The heat that the materials are subjected as they are conveyed through the extruder causes the polymer to melt to thereby facilitate thorough homogenization of all of the ingredients. The radiopaque agent powder, selected for its uniform particle shape and controlled particle size distribution as described above is subsequently introduced into the melt stream via a secondary feeder, again at a carefully controlled mass flow rate so as to achieve the target fill ratio. The solid powder, molten polymer and additives are homogenized as they are conveyed downstream and discharged through a die as molten strands which are cooled in water and subsequently pelletized. The preferred extrusion equipment employs two independent feeders as introduction of all components through a single primary feeder would require significantly higher machine torques and result in excessive screw and barrel wear. The powder feeder is preferentially operated in tandem with a sidefeeder device, which in turn conveys the powder through a sealed main barrel port directly into the melt stream. A preferred composition comprises a fill ratio of 91.3 weight percent of tungsten (H.C. Starck's Kulite HC600s, HC180s and KMP-103JP) to Pebax 40D. A maleic anhydride source in the form of Licomont AR504 is initially added to the polymer resin at the rate of approximately 3 pphr while an antioxidant in the form of Ciba Geigy B225 at the rate of approximately 2 pphr (parts per hundred relative to the resin). The temperature to which materials are subjected to in the extruder is about 221°C.

Once the marker material has been compounded, the marker can be fabricated in suitable dimensions by an extrusion coating process. While free extrusion is possible, this method is problematic due to the high fill ratios of the polymeric materials. Extrusion onto a continuous length of beading has been found to lend the necessary support for the molten extrudate to prevent breakage. The support beading may take the form of a disposable, round mandrel made of PTFE, teflon coated stainless steel wire or other heat resistant material that does not readily bond to the extrudate. By additionally limiting the area draw down ratio (ADDR) to below 10:1 the tungsten-laden melt can successfully be drawn to size by an extrusion puller. The beading provides the added benefit of fixing the inner diameter and improving overall dimensional stability of the final tungsten/polymer coating. Extrusion of the 91.3 weight percent fill ratio tungsten/Pebax composition described above over 0.5461mm (0.0215") diameter PTFE beading were successfully drawn down to a wall thickness of 0.0635mm (0.0025") to yield a marker properly sized for attachment to for example a 0.559mm (0.022") diameter inner member of balloon catheter. Also, extrusion coatings of 91% compound over 0.178mm (0.007") Teflon coated stainless steel wire were successfully drawn down to single wall thicknesses of 0.051mm (0.002") to make guide wire coatings.

Once the extrudate has cooled, the extrusion is simply cut to the desired lengths (e.g., 1 to 1.5 mm) of the individual markers, such as with the use of a razor blade and reticle, preferably with the beading still in place to provide support during cutting. The beading remnant is subsequently ejected and the marker is slipped onto a medical device or a particular component thereof Finally, the marker is attached to the underlying substrate, preferably with the use of heat shrink tubing and a heat source (hot air, laser, etc.) wherein the heat (∼171-210°C) simultaneously causes the marker to melt and the heat shrink tubing to exert a compressive force on the underlying molten material. Heat bonding a marker, onto an underlying component provides the added benefit of slightly tapering the edges of the marker to reduce the likelihood of catching an edge and either damaging the marker or the medical device during assembly or handling of the medical device.

A marker formed as per the above described compounding, fabricating and assembling processes, having a fill ratio of 91.3 weight percent (36.4 volume percent) with a wall thickness of 0.0635mm (0.0025") has been shown to have dramatically more radiopacity than commercially available 80 weight percent compounds and comparable to the radiopacity of 0.03175mm (0.00125") thick conventional Platinum/10% Iridium markers.

In various alternative embodiments, the polymer markers formed as per the above-described processes and having a fill ratio of up about 95 weight percent have exhibited even higher radiopacities for a given size. It is preferable that the fill ratio be in the range of about 91 weight percent or more up to about 95 weight percent, and including anything therebetween. Advantageously, these higher weight percent radiopaque material loadings allow the markers to be made into a smaller cross-sectional size while maintaining the same level of radiopacity of a larger marker. A smaller yet highly radiopaque marker reduces the crossing profile, and minimizes its effect on the flexibility of the stent, catheter, guide wire, or similar device to which it is attached or mounted. Accordingly, the radiopaque metal bands, electroplated gold bands, or radiopaque coils whose inherent high modulus of elasticity affected the flexibility of prior art guide wires, for example, can be replaced by the polymer markers of the present invention.

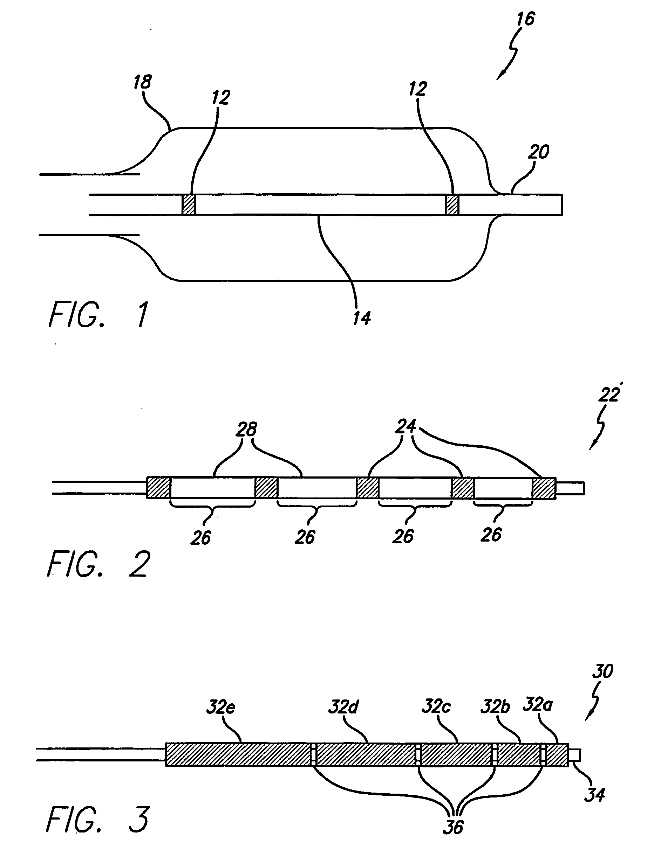

Figure 1 illustrates two radiopaque markers 12 attached to the inner member 14 of a balloon catheter 16. The markers attached to the inner member prior to the positioning of the inner member within the balloon 18 and attachment thereto at 20. Fluoroscopic illumination of the device allows the invisible balloon to be positioned relative to a lesion by virtue of the visibility of the radiopaque markers and their known positions relative to the balloon.

Figure 2 illustrates a preferred embodiment of a guide wire with a measurement feature 22 wherein a series of radiopaque markers 24 are attached to the guide wire 22 at preselected separation distances 26 allow the device to be used as a type of ruler to measure the size of a lesion. The separation between adjacent markers may be controlled by the use of a radiotransparent tubular spacers 28 that are similarly melt bondable to the underlying guide wire. Upon assembly of the radiopaque markers and the radiotransparent spacers onto the guide wire, heat shrink tubing of sufficient length is slipped over the entire section of guide wire and heated to the appropriate temperature to cause both the markers as well as the spacers to become melt bonded to the guide wire.

Figure 3 illustrates an alternatively preferred embodiment of a guide wire with a measurement feature 30 wherein an equally spaced series of differently sized radiopaque markers 32a-e are attached to a guide wire 34 to allow the device to be used to gauge the size of a lesion. The separation between adjacent markers may be controlled by the use of a radiotransparent tubular spacers 36 that are similarly melt bondable to the underlying guide wire. Upon assembly of the radiopaque markers and the radiotransparent spacers onto the guide wire, heat shrink tubing of sufficient length is slipped over the entire section of guide wire and heated to the appropriate temperature to cause both the markers as well as the spacers to become melt bonded to the guide wire.

The present invention radiopaque polymer bands provide the same or better radiopacity than platinum-iridium bands or coils. As mentioned above, radiopaque polymer is advantageous over metal bands due to the flexibility of the polymer versus metal. The polymer's low elastic modulus allows the guide wire to bend along its natural path without interference or inducing kinking. Also of significant benefit is the lower cost of using radiopaque polymer as compared to the conventional use of precious metal (i.e., gold, platinum, etc.) marker system.

One method of manufacturing the above-described guide wires is through extrusion coating. Specifically, the radiopaque polymer is extrusion coated over a PTFE mandrel. The resultant radiopaque polymer coating in its cylindrical form is then cut to the desired discrete lengths and removed from the mandrel. These discrete polymer lengths take the form of slugs or bands, which can be individually positioned over the guide wire and melt-bonded to the wire core at the desired locations and/or at the predetermined interval spacings.

Next, the guide wire may optionally have a separate polymer coating over the length of the portion of the shaft that covers the markers, thus providing a smoother, all-polymeric outside diameter. This outer coating overlying the markers can be between about 0.0127mm to about 0.127mm (about 0.0005" to about 0.005") in wall thickness, and is preferably between about 0.0254mm to about 0.0508mm (about 0.001" to about 0.002") in wall thickness.

The outer coating can be applied to the guide wire through different methods. One method is through a polymer shrink tube of polytetrafluorethylene (PTFE), fluorinated ethylene polymer (FEP), hydroxy-terminated polyether (HTPE), or some other flexible, heat shrinkable polymer. The shrink tube is placed over a portion or all of the wire and the polymer markers, and the shrink tube is then heated. Another coating method is through a polymer extrusion technique such as that shown in for example, U.S. Patent No. 6,419,745 (Burkett, et al.). In this process, a guide wire and a polymer cartridge are extruded simultaneously through a die. The polymer cartridge is melted just before being advanced through the die with the guide wire, resulting in an even coating covering the wire. Yet another method is through dip coating the guide wire with its polymer markers. After its application to the wire core, the outer coating may then serve as a substrate for the addition thereon of a hydrophilic, silicone-based coating, or other lubricious-type coating.

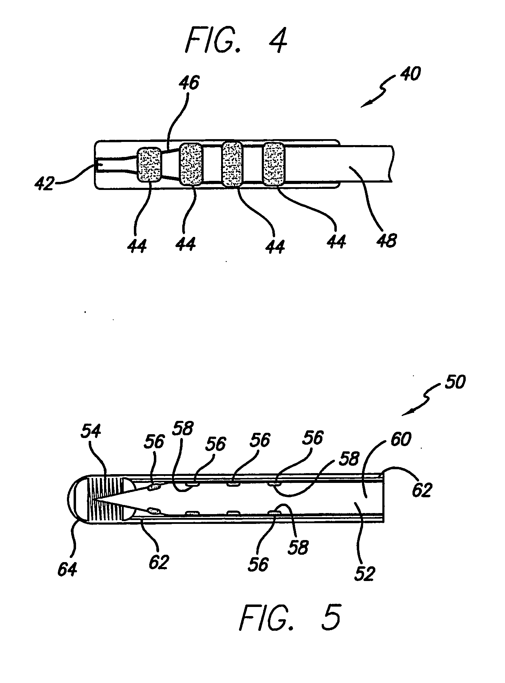

In one preferred embodiment shown in a simplified, partial side elevational view of Figure 4, a guide wire 40 is covered at the distal portion 48 with radiopaque polymer markers 44 that are covered by a polymer coating 46 and/or other type of polymer cover, shown in cross-section. The radiopaque markers 44 are optionally made from a polymer doped with a radiographically dense element such as tungsten powder. The tungsten powders is preferably doped at about 91-93 weight percent, but can be as high as about 95 weight percent, to provide desired radiopacity. As seen in Figure 4, the polymer-coated section is preferably located at the distal portion 48 of the guide wire 40, about 3-7 cm from the distal tip 42 of the guide wire 40. The radiopaque polymer markers 44 have a hollow cylindrical form and are preferably about 1-2 mm in length and spaced apart by predetermined distances. In various embodiments, the radiopaque polymer markers 44 can range from about 0.5 mm to about 5 mm in length, where the preferred dimension is about 2 mm in length. The inside diameter of the radiopaque polymer markers 44 is preferably from about 0.076mm to about 0.254mum (about 0.003" to about 0.010"). The preferred thickness of the marker 44 is about 0.127mm to about 0.203mm (about 0.005" to about 0.008"). The outside diameter may be from about 0.254mm to about 0.356mm (about 0.010" to about 0.014") where the preferred outside diameter is about 0.305mm (about 0.012").

Such an arrangement (i.e., markers at about 3-7 cm from distal tip) provides markers 44 of sufficient radiopacity (about 91-93 weight percent doping) yet compact dimensions (about 1-2mm length and about 0.127-0.203mem (0.005-0.008 inch) thickness) on a guide wire that can be used for measuring lesions inside the coronary anatomy, aiding in stent deployment, balloon angioplasty, and other medical procedures. Furthermore, such dimensions are selected so that the polymer markers 44 have sufficient radiopacity yet do not generally interfere with the flexibility, torqueability, or other performance qualities of the guide wire.

In the Figure 4 embodiment, the radiopaque polymer markers 44 optionally extend above the surface of the core creating a slightly uneven surface profile. There is, however, an optional polymer coating 46 that is sufficiently thick which fills in the uneven surface profile; by doing so, the polymer coating 46 leaves a generally smooth outside diameter surface profile. This improves trackability of the guide wire, meaning that the ease of movement of catheters or other devices over the wire is improved. This outside diameter profile may follow the taper or shape of the wire core, or as depicted in Figure 4, it may be blunt, while the core has a pointed taper, so as not to follow the shape of the core.

Figure 5 is a simplified, partial cross-sectional view of the distal portion 52 of an alternative embodiment guide wire 50. The wire 50 has a tip coil 54. Some of the radiopaque polymer markers 56 are optionally recessed into respective grooves 58 formed into the surface of the core 60 to allow the markers 56 to sit relatively flush with the outer surface of the core 60 as depicted in Figure 5. The recessed radiopaque polymeric markers 56 provide a generally smooth outside diameter and can improve trackability. A PTFE heat shrink sleeve 62 is optionally applied over the top of the polymer markers 56, while the tip coil 54 in this exemplary embodiment is uncovered and exposed.

As seen in the embodiment illustrated in Figure 5 and described above, the radiopaque polymer markers 56 may be placed on the bare metal core of the guide wire 50, for example. The markers 56 are attached to the core by gluing, melting, or any method known in the art to keep the markers in place. In one alternative embodiment, the core includes a liquid polymer (replacing the heat shrink sleeve 62) added to the surface of the core, which polymer would be cured by, for example, ultraviolet light or heat. Alternatively, the liquid polymer could be added by dipping the core into a pool or liquid polymer, or the liquid polymer could be manually applied by brushing, spraying, etc. The end result is a uniform and smooth exterior polymeric surface covering the markers, which markers themselves may or may not be flush with the surface profile of the wire core. The liquid polymer coating covering the radiopaque markers can be made to a wall thickness of preferably about 0.0127mm to 0.076mm (about 0.0005" to 0.003"). This range of thicknesses provides sufficient protection for the markers, gives a smooth outside surface, and has enough bulk to serve as a substrate for additional top coatings if desired.

As to additional top coatings, the guide wire 50 may optionally have a separate, outer polymer cover 64 overlying at least part of the length of the shaft and that covers the liquid polymer surface to provide an even smoother, all-polymeric outside diameter. This outer polymer cover 64 may also act as a substrate for a hydrophilic, silicone-based, or other lubricious coating (not shown). In one embodiment, the outer polymer cover 64 is a urethane doped with a radiopaque or radiographically dense element such as tungsten powder. Other doping particles are also contemplated, such as platinum, gold, iridium, palladium, rhenium, rhodium, tantalum, silver and tin. Such a guide wire 50 would then have both radiopaque polymer markers 56 and a radiopaque outer polymer cover 64. The level of radiopacity of the outer polymer cover 64 may of course be adjusted by the amount of doping of the radiographically dense element. The fill ratio of the radiographically dense element relative to the base polymer may range from a few weight percent up to about 95 weight percent and including everything therebetween.

In sum, either or both the outer polymeric cover 64 and the polymeric markers 56 can be made to be radiopaque. The markers 56 in one embodiment take the form of bands or rings, while the cover 64 has much greater length and may cover the distal tip as seen in Figure 5. Use of such radiopaque polymeric structures on a medical device would obviate the need for conventional marker bands made from precious metals such as gold or platinum. From a design standpoint, the polymeric radiopaque structures as described can take many forms and thus are much more versatile than precious metals which are difficult and expensive to fabricate.

In various alternative embodiments, the highly radiopaque polymer markers can be applied to different intraluminal medical devices aside from a guide wire. Such intraluminal medical devices include but are not limited to a balloon, an embolic filter, and a stent The intraluminal medical device functions as a substrate on which at least one polymeric radiopaque marker is disposed. Means for joining the marker to the substrate are similar to that already discussed above. The polymeric radiopaque marker includes a radiopaque material or particle, as described above, such as tungsten powder in an amount greater than about 91 weight percent, and preferably about 91-93 weight percent, of the marker. A polymer coating may optionally overlie the marker or markers.

While a particular form of the invention has been described, it will be apparent to those skilled in the art that various modifications can be made without departing from the scope of the invention. More specifically, a variety of different polymers and radiopaque agents can be compounded using the appropriate wetting agent, markers of different shape and dimensions can be formed and the markers can be attached to any of a variety of medical devices that can benefit from being radiopaquely marked. Accordingly, it is not intended that the invention be limited except by the appended claims.

## Claims

1. A radiopaque marker, comprising:
a polymer;
radiopaque particles disposed within the polymer having an average diameter of at least about 2 microns and a maximum diameter of about 20 microns; and
a wetting agent including pelletized MA-g-po for facilitating encapsulation of the particles by the polymer, wherein the radiopaque particles include greater than about 91 weight percent of the marker.

2. The radiopaque marker of claim 1, wherein the radiopaque particles comprise about 91-95 weight percent of the marker.

3. The radiopaque marker of claim 1, wherein the polymer is selected from a group consisting of Pebax, polyetherurethanes, polyester copolymers, olefin derived copolymers, natural rubbers, synthetic rubbers, thermoplastic elastomers, specialty polymers, polyurethanes, and nylon.

4. The radiopaque marker of claim 1, wherein the radiopaque particles are selected from a group consisting of platinum, gold, iridium, palladium rhenium, rhodium, tungsten, tantalum, silver and tin.

5. The radiopaque marker of claim 2, wherein the marker includes a tubular form.

6. A method of marking a medical device with a radiopaque material, comprising:
providing a flexible polymeric radiopaque marker containing pelletized MA-g-PO and greater than 91 weight percent radiopaque particles, wherein such particles have an average diameter of at least about 2 microns and a maximum diameter of about 20 microns;
positioning the marker on the medical device; and
melt bonding the marker in place.

7. The method of claim 6, wherein the radiopaque particles comprise about 91-95 weight percent of the marker.

8. The method of claim 6, wherein the polymer is selected from a group consisting of Pebax, polyetheruethanes, polyester copolymers, olefin derived copolymers, natural rubbers, synthetic rubbers, thermoplastic elastomers, specialty polymers, polyurethanes and nylon.

9. The method of claim 6, wherein the radiopaque particles are chosen from a group consisting of platinum, gold, iridium, palladium, rhenium, rhodium, tungsten, tantalum, silver, and tin.

10. The method of claim 7, wherein the marker is formed on a PTFE mandrel.

11. The method of claim 10, wherein the markers are equally spaced relative to one another so as to function as a ruler under fluoroscopic inspection.

12. An intracorporeal guide wire, comprising:
a wire core having a distal section and a proximal section;
at least one polymeric radiopaque marker disposed at the distal section, wherein the marker includes pelletized MA-g-PO and a radiopaque material in an amount greater than about 91 weight percent of the marker wherein said radiopaque material includes particles having an average diameter of at least about 2 microns and a maximum diameter of about 20 microns; and
a polymer coating overlying the marker.

13. The guide wire of claim 12, wherein the radiopaque material includes about 91-93 weight percent inclusive of the marker.

14. The guide wire of claim 12, wherein the radiopaque particles are selected from a group consisting of platinum, gold, iridium, palladium, rhenium, rhodium, tungsten, tantalum, silver, and tin.

15. The guide wire of claim 12, wherein the marker and at least the distal section is coated with a second polymer.

16. The guide wire of claim 12, wherein the wire core at least in the distal section includes a groove receiving the marker therein such that an exterior surface of the marker is relatively flush with the exterior of the wire core.

17. The guide wire of claim 12, wherein the polymer coating includes heat shrink tubing of at least one of PTFE, FEP, and HTPE.

18. The guide wire of claim 12, wherein the radiopaque material includes tungsten that is about 91-93 weight percent inclusive of the marker.

19. The guide wire of claim 12, wherein a hydrophilic coating is disposed over the polymer coating.

20. The guide wire of claim 12, wherein the guide wire includes a plurality of relatively equally spaced apart markers.

21. The guide wire of claim 12, wherein the polymer coating includes a liquid polymer cured by UV or heat, and an outer polymer cover at least partially overlying the liquid polymer that is doped with a radiopaque material

22. The guide wire of claim 12, wherein the radiopaque marker creates an uneven surface contour on the guide wire and the polymer coating overlying the radiopaque marker fills in the uneven surface contour to create a smooth exterior.

23. An intraluminal medical device, comprising;
a substrate structure selected from the group consisting of a guide wire, a balloon, an embolic filter, and a stent;
at least one polymeric radiopaque marker disposed on the substrate structure, wherein the marker includes pelletized MA-g-PO and a radiopaque material in an amount greater than about 91 weight percent of the marker wherein said radiopaque material includes particles having an average diameter of at least about 2 microns and a maximum diameter of about 20 microns; and
a polymer coating overlying the marker.

## Patentansprüche

1. Radiopaker Marker, umfassend
ein Polymer;
radiopake Teilchen, die innerhalb des Polymers angeordnet sind, mit einem mittleren Durchmesser von mindestens etwa zwei Mikrometer und einem maximalen Durchmesser von etwa 20 Mikrometer; und
ein Benetzungsmittel, enthaltend pelletiertes MA-gPO zum Erleichtern der Einkapselung der Teilchen durch das Polymer, worin die radiopaken Teilchen mehr als etwa 91 Gewichtsprozent des Markers umfassen.

2. Radiopaker Marker nach Anspruch 1, worin die radiopaken Teilchen etwa 91-95 Gewichtsprozent des Markers umfassen.

3. Radiopaker Marker nach Anspruch 1, worin das Polymer ausgewählt ist aus einer Gruppe, bestehend aus Pebax, Polyetherurethanen, Polyestercopolymeren, von Olefin abgeleiteten Copolymeren, natürlichen Gummis, synthetischen Gummis, thermoplastischen Elastomeren, Spezialpolymeren, Polyurethanen und Nylon.

4. Radiopaker Marker nach Anspruch 1, worin die radiopaken Teilchen ausgewählt sind aus einer Gruppe, bestehend aus Platin, Gold, Iridium, Palladium, Rhenium, Rhodium, Wolfram, Tantal, Silber und Zinn.

5. Radiopaker Marker nach Anspruch 2, worin der Marker eine schlauchförmige Ausgestaltung umfasst.

6. Verfahren zum Herstellen einer medizinischen Vorrichtung mit einem radiopaken Material, umfassend:
Bereitstellen eines flexiblen polymeren radiopaken Markers, enthaltend pelletiertes MA-g-PO und mehr als 91 Gewichtsprozent radiopake Teilchen, worin solche Teilchen einen mittleren Durchmesser von mindestens etwa zwei Mikrometer und einen maximalen Durchmesser von etwa 20 Mikrometer aufweisen;
Positionieren des Markers auf der medizinischen Vorrichtung; und
Schmelzbinden des Markers an der Stelle.

7. Verfahren nach Anspruch 6, worin die radiopaken Teilchen etwa 91-95 Gewichtsprozent des Markers umfassen.

8. Verfahren nach Anspruch 6, worin das Polymer ausgewählt wird aus einer Gruppe, bestehend aus Pebax, Polyetherurethanen, Polyestercopolymeren, von Olefin abgeleiteten Copolymeren, natürlichen Gummis, synthetischen Gummis, thermoplastischen Elastomeren, Spezialpolymeren, Polyurethanen und Nylon.

9. Verfahren nach Anspruch 6, worin die radiopaken Teilchen ausgewählt werden aus einer Gruppe, bestehend aus Platin, Gold, Iridium, Palladium, Rhenium, Rhodium, Wolfram, Tantal, Silber und Zinn.

10. Verfahren nach Anspruch 7, worin der Marker auf einem PTFE-Dorn gebildet wird.

11. Verfahren nach Anspruch 10, worin die Marker relativ zueinander in gleichem Abstand sind, um als ein Maßstab unter fluoroskopischer Untersuchung zu wirken.

12. Intracorporaler Führungsdraht, umfassend:
einen Draht mit einem distalen Abschnitt und einem proximalen Abschnitt;
mindestens einen radiopaken Marker, der an dem distalen Abschnitt angeordnet ist;
worin der Marker pelletiertes MA-g-PO und ein radiopakes Material in einer Menge von mehr als etwa 91 Gewichtsprozent des Markers umfasst, worin das radiopake Material Teilchen mit einem mittleren Durchmesser von mindestens etwa zwei Mikrometer und einem maximalen Durchmesser von etwa 20 Mikrometer umfasst; und
eine Polymerbeschichtung, die den Marker bedeckt.

13. Führungsdraht nach Anspruch 12, worin das radiopake Material etwa 91-93 Gewichtsprozent inklusive des Markers umfasst.

14. Führungsdraht nach Anspruch 12, worin die radiopaken Teilchen ausgewählt sind aus einer Gruppe, bestehend aus Platin, Gold, Iridium, Palladium, Rhenium, Rhodium, Wolfram, Tantal, Silber und Zinn.

15. Führungsdraht nach Anspruch 12, worin der Marker und mindestens der distale Abschnitt mit einem zweiten Polymer beschichtet ist.

16. Führungsdraht nach Anspruch 12, worin der Drahtkern zumindest im distalen Abschnitt eine Vertiefung enthält, die den Marker aufnimmt, sodass eine äußere Oberfläche des Markers relativ bündig mit dem Äußeren des Drahtkerns ist.

17. Führungsdraht nach Anspruch 12, worin die Polymerbeschichtung einen Schrumpfschlauch aus mindestens einem aus PTFE, FEP und HTPE umfasst.

18. Führungsdraht nach Anspruch 12, worin das radiopake Material Wolfram umfasst, das etwa 91-93 Gewichtsprozent inklusive des Markers ist.

19. Führungsdraht nach Anspruch 12, worin eine hydrophile Beschichtung über der Polymerbeschichtung angeordnet ist.

20. Führungsdraht nach Anspruch 12, worin der Führungsdraht mehrere Marker umfasst, die zueinander in gleichem Abstand sind.

21. Führungsdraht nach Anspruch 12, worin die Polymerbeschichtung ein flüssiges Polymer, gehärtet durch UV oder Wärme, und eine äußere Polymerbeschichtung, die zumindest teilweise das flüssige Polymer bedeckt, das mit einem radiopaken Material dotiert ist, umfasst.

22. Führungsdraht nach Anspruch 12, worin der radiopake Marker eine ungleichmäßige Oberflächenkontur auf dem Führungsdraht bildet und die Polymerbeschichtung, die den radiopaken Marker bedeckt, die ungleichmäßige Oberflächenkontur füllt, um eine glatte Außenseite zu bilden.

23. Intraluminale medizinische Vorrichtung, umfassend:
eine Substratstruktur, ausgewählt aus der Gruppe, bestehend aus einem Führungsdraht, einem Ballon, einem embolischen Filter und einem Stent;
mindestens einen polymeren radiopaken Marker, der auf der Substratstruktur angeordnet ist;
worin der Marker pelletiertes MA-g-PO und ein radiopakes Material in einer Menge von mehr als etwa 91 Gewichtsprozent des Markers umfasst, worin das radiopake Material Teilchen mit einem mittleren Durchmesser von mindestens etwa zwei Mikrometer und einem maximalen Durchmesser von etwa 20 Mikrometer umfasst; und
eine Polymerbeschichtung, die den Marker bedeckt.

## Revendications

1. Marqueur radio-opaque, comprenant :
un polymère ;
des particules radio-opaques présentes dans le polymère, ayant un diamètre moyen d'au moins environ 2 micromètres et un diamètre maximal d'environ 20 micromètres ; et
un agent mouillant comprenant du MA-g-PO granulé pour faciliter l'encapsulation des particules par le polymère, dans lequel les particules radio-opaques représentent plus d'environ 91 pour cent en poids du marqueur.

2. Marqueur radio-opaque suivant la revendication 1, dans lequel les particules radio-opaques représentent environ 91 à 95 pour cent en poids du marqueur.

3. Marqueur radio-opaque suivant la revendication 1, dans lequel le polymère est choisi dans le groupe consistant en le Pebax, des polyétheruréthannes, des copolymères du type polyester, des copolymères dérivés d'oléfines, des caoutchoucs naturels, des caoutchoucs synthétiques, des élastomères thermoplastiques, des polymères spéciaux, des polyuréthannes et un Nylon.

4. Marqueur radio-opaque suivant la revendication 1, dans lequel les particules radio-opaques sont choisies dans le groupe consistant en platine, or, iridium, palladium, rhénium, rhodium, tungstène, tantale, argent et étain.

5. Marqueur radio-opaque suivant la revendication 2, le marqueur comprenant une forme tubulaire.

6. Procédé pour marquer un dispositif médical avec une matière radio-opaque, comprenant les étapes consistant :
à fournir un marqueur radio-opaque polymère flexible contenant du MA-g-PO granulé et une proportion supérieure à 91 pour cent en poids de particules radio-opaques, ces particules ayant un diamètre moyen d'au moins environ 2 micromètres et un diamètre maximal d'environ 20 micromètres ;
à positionner le marqueur sur le dispositif médical et à lier par fusion le marqueur en place.

7. Procédé suivant la revendication 6, dans lequel les particules radio-opaques représentent environ 91 à 95 pour cent en poids du marqueur.

8. Procédé suivant la revendication 6, dans lequel le polymère est choisi dans le groupe consistant en Pebax, des polyétheruréthannes, des copolymères du type polyester, des copolymères dérivés d'oléfines, des caoutchoucs naturels, des caoutchoucs synthétiques, des élastomères thermoplastiques, des polymères spéciaux, des polyuréthannes et un Nylon.

9. Procédé suivant la revendication 6, dans lequel les particules radio-opaques sont choisies dans le groupe consistant en platine, or, iridium, palladium, rhénium, rhodium, tungstène, tantale, argent et étain.

10. Procédé suivant la revendication 7, dans lequel le marqueur est formé sur un mandrin en PTFE.

11. Procédé suivant la revendication 10, dans lequel les marqueurs sont espacés de manière égale les uns par rapport aux autres de manière à jouer le rôle de règle en examen fluoroscopique.

12. Fil de guidage intracorporel, comprenant :
une âme de fil ayant une section distale et une section proximale ;
au moins un marqueur radio-opaque polymère placé à la section distale, le marqueur comprenant du MA-g-PO granulé et une matière radio-opaque en une quantité supérieure à environ 91 pour cent en poids du marqueur, ladite matière radio-opaque comprenant des particules ayant un diamètre moyen d'au moins environ 2 micromètres et un diamètre maximal d'environ 20 micromètres ; et
un revêtement polymère recouvrant le marqueur.

13. Fil de guidage suivant la revendication 12, dans lequel la matière radio-opaque représente environ 91 à 93 pour cent en poids inclus du marqueur.

14. Fil de guidage suivant la revendication 12, dans lequel les particules radio-opaques sont choisies dans le groupe consistant en platine, or, iridium, palladium, rhénium, rhodium, tungstène, tantale, argent et étain.

15. Fil de guidage suivant la revendication 12, dans lequel le marqueur et au moins la section distale sont revêtus d'un second polymère.

16. Fil de guidage suivant la revendication 12, dans lequel l'âme du fil au moins dans la section distale comprend une rainure recevant le marqueur dans celle-ci de telle sorte que la surface extérieure du marqueur soit relativement au ras de l'extérieur de l'âme du fil.

17. Fil de guidage suivant la revendication 12, dans lequel le revêtement polymère comprend une tubulure thermo-rétractable d'au moins un des PTFE, FEP et HTPE.

18. Fil de guidage suivant la revendication 12, dans lequel la matière radio-opaque comprend du tungstène représentant environ 91 à 93 pour cent en poids inclus du marqueur.

19. Fil de guidage suivant la revendication 12, dans lequel un revêtement hydrophile est placé sur le revêtement polymère.

20. Fil de guidage suivant la revendication 12, ledit fil de guidage comprenant une pluralité de marqueurs également espacés les uns des autres.

21. Fil de guidage suivant la revendication 12, dans lequel le revêtement polymère comprend un polymère liquide durci par le rayonnement UV ou la chaleur, et une couverture polymère extérieure recouvrant au moins partiellement le polymère liquide, qui est dopée avec une matière radio-opaque.

22. Fil de guidage suivant la revendication 12, dans lequel le marqueur radio-opaque crée un contour de surface irrégulier sur le fil de guidage et le revêtement polymère recouvrant le marqueur radio-opaque remplit le contour de surface irrégulier pour créer une surface extérieure lisse.

23. Dispositif médical intraluminal, comprenant:
une structure de substrat choisie dans le groupe consistant en un fil de guidage, un ballonnet, un filtre embolique et un extenseur ;
au moins un marqueur radio-opaque polymère placé sur la structure de substrat, ledit marqueur comprenant du MA-g-PO granulé et une matière radio-opaque en une quantité supérieure à environ 91 pour cent en poids du marqueur, ladite matière radio-opaque comprenant des particules ayant un diamètre moyen d'au moins environ 2 micromètres et un diamètre maximal d'environ 20 micromètres ; et
un revêtement polymère recouvrant le marqueur.
